(19) Europäisches Patentamt

European Patent Office

Office européen des brevets

(11) EP 0 823 249 B1

## (12) FASCICULE DE BREVET EUROPEEN

(45) Date de publication et mention de la délivrance du brevet:
**14.07.1999 Bulletin 1999/28**

(51) Int. Cl.⁶: $A61K\ 7/00$, $A61K\ 7/48$, $A61K\ 7/42$

(21) Numéro de dépôt: **97401495.3**

(22) Date de dépôt: **26.06.1997**

(54) **Composition de protection de la peau ou des cheveux contenant des nanopigments et un organopolysiloxane solide élastomère associé à une phase grasse**

Zusammensetzung zum Schutz von Haut oder Haaren, die Nanopigmente und ein festes Organopolysiloxan-Elastomer in einer Fettphase enthält

Composition for the protection of the skin or hair containing nanopigments and solid organopolysiloxan elastomer in a fatty phase

(84) Etats contractants désignés:
**DE ES FR GB IT**

(30) Priorité: **29.07.1996 FR 9609525**

(43) Date de publication de la demande:
**11.02.1998 Bulletin 1998/07**

(73) Titulaire: **L'OREAL**
**75008 Paris (FR)**

(72) Inventeurs:
 • **Bara, Isabelle**
 **75013 Paris (FR)**
 • **Lemann, Patricia**
 **92320 Chatillon (FR)**

(74) Mandataire: **Lhoste, Catherine**
**L'OREAL,**
**D.P.I.,**
**6, rue Bertrand Sincholle**
**92585 Clichy Cédex (FR)**

(56) Documents cités:
**EP-A- 0 271 925**    **EP-A- 0 279 319**
**EP-A- 0 295 886**    **EP-A- 0 456 459**
**EP-A- 0 545 002**    **FR-A- 2 646 346**
**FR-A- 2 680 684**

**Description**

La présente demande se rapporte à une composition à application topique contenant des nanopigments et un organopolysiloxane solide élastomère associé à une phase grasse, en vue d'améliorer l'indice de protection dans le rayonnement ultraviolet, apporté par les nanopigments. Elle se rapporte aussi à l'utilisation d'un organopolysiloxane solide élastomère associé à une phase grasse, dans et/ou pour la fabrication d'une composition à application topique pour le soin, le traitement ou le maquillage de la peau ou pour le soin des cheveux, contenant des nanopigments et ayant des propriétés filtrantes élevées. Cette composition peut être appliquée sur le visage, le corps et/ou les jambes d'êtres humains ainsi que sur les mains et le cuir chevelu.

On sait que les rayons lumineux de longueurs d'onde comprises entre 280 nm et 400 nm permettent le brunissement de l'épiderme humain et que, par ailleurs, les rayons de longueurs d'onde comprises entre 280 nm et 320 nm, connus sous la dénomination d'UVB, provoquent des érythèmes et des brûlures cutanées qui peuvent nuire au développement du bronzage naturel ; ce rayonnement UVB doit donc être filtré.

On sait également que les rayons UVA, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UVA provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UVA.

Pour filtrer les rayons UVA et UVB, il existe sur le marché différents types de filtres solaires : les pigments et les filtres chimiques. Ces filtres solaires doivent pouvoir absorber ou bloquer les rayons nocifs du soleil tout en restant inoffensifs pour l'utilisateur.

A ce titre, les pigments d'oxydes métalliques sont de plus en plus utilisés dans les produits solaires et les produits de jour, y compris de maquillage, compte tenu de leurs propriétés de diffusion et de réflexion des rayonnements UV qui leur confèrent un grand intérêt en terme de photoprotection : utilisés seuls, ils permettent d'obtenir une bonne protection contre les rayons UV ; associés à des filtres chimiques organiques, ils permettent de réaliser des produits hautement photoprotecteurs. Ces pigments sont utilisés de préférence sous forme de nanopigments, dont les propriétés filtrantes sont bien connues.

Les médecins se rendent compte de plus en plus que les rayons solaires provoquent des réactions pouvant entraîner des maladies graves comme le cancer de la peau. Aussi, on cherche de plus en plus à faire des produits solaires ainsi que des produits de maquillage ou de soin ayant de hauts indices de protection.

Il est connu du document FR-A-2 680 684 des compositions comprenant des nanopigments et des polymères à structure siloxanique porteurs de groupement absorbant les ultraviolets.

Pour des raisons de stabilité de ces compositions et/ou de toxicité sur la peau ou les muqueuses, il n'est pas toujours possible d'utiliser des filtres chimiques ou d'augmenter leurs quantités pour augmenter l'indice de protection des compositions contenant des nanopigments. Aussi la demanderesse a cherché un autre moyen pour augmenter l'indice de protection de ces compositions.

De façon surprenante, la demanderesse a trouvé qu'en associant les nanopigments avec un organopolysiloxane solide élastomère associé à une phase grasse, on augmentait le pouvoir filtrant des nanopigments et que, de ce fait, on améliorait nettement l'indice de protection (IP) de la composition les contenant.

Aussi, l'invention a pour objet une composition à application topique contenant des nanopigments d'au moins un oxyde métallique, la taille moyenne des particules élémentaires desdits nanopigments allant de 5 à 100 nm, caractérisée en ce qu'elle contient en outre un organopolysiloxane solide élastomère associé à une phase grasse, en vue d'améliorer l'indice de protection apporté par les nanopigments.

Certes, le document EP-A-545002 décrit des compositions cosmétiques contenant un organopolysiloxane solide élastomère associé à une phase grasse, et susceptible de contenir des pigments. Toutefois, ce document ne décrit pas l'association d'un tel organopolysiloxane avec des nanopigments. En outre, il ne décrit ni ne suggère que l'organopolysiloxane puisse augmenter ainsi la protection solaire apportée par les nanopigments.

Il est connu que les nanopigments présentent l'inconvénient d'être difficiles à introduire dans les compositions cosmétiques ou dermatologiques, du fait de leur petite taille. En effet, la taille moyenne des particules élémentaires de nanopigments s'échelonne de 5 à 100 nm et de préférence de 10 à 60 nm, ce qui représente des surfaces spécifiques dix fois supérieures à celles des pigments classiques. De ce fait, les nanopigments sont difficiles à disperser. Par ailleurs, les nanopigments ont tendance à déstabiliser les émulsions les contenant et à s'agglomérer au cours du temps ou lors de l'étalement sur la peau, de la composition les contenant.

En plus de l'augmentation de l'indice de protection, l'utilisation d'un organopolysiloxane solide élastomère selon l'invention permet d'obtenir une composition contenant des nanopigments, qui est stable et dans laquelle les nanopigments sont parfaitement dispersés, ce bon état de dispersion étant maintenu dans le temps, à la fois dans la composition et après étalement sur la peau ou les cheveux.

Aussi, l'invention a encore pour objet l'utilisation d'un organopolysiloxane solide élastomère associé à une phase grasse dans une composition à application topique contenant des nanopigments, la taille moyenne des particules élémentaires desdits nanopigments allant de 5 à 100 nm, pour améliorer la stabilité de la dite composition et/ou conférer à la dite composition un indice de protection amélioré.

En outre, l'organopolysiloxane solide élastomère selon l'invention confère à la composition de meilleures qualités sensorielles d'étalement et permet l'obtention d'un effet mat et waterproff.

L'indice de protection (IP) représente le pouvoir filtrant dans le domaine des UVA. La détermination de cet indice de protection UVA est basée sur la méthode d'évaluation de la pigmentation immédiate et persistante induite par les UVA (Persistant Pigment Darkening : PPD), décrite par Chardon et col. (Method for the UVA protection assessment of sunscreens based on residual immediate pigment darkening. 20th Annual Meeting of the American Society for Photobiology, Marco island, Florida (USA), June 20-24, 1992).

Les organopolysiloxanes élastomères associés à une phase grasse conformes à l'invention sont en général partiellement ou totalement réticulés et éventuellement de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées.

Les organopolysiloxanes élastomères associés à une phase grasse de l'invention se présentent généralement sous forme de gel constitué d'un organopolysiloxane élastomère associé à une phase grasse, inclus dans au moins une huile hydrocarbonée et/ou une huile siliconée. Ils peuvent être choisis notamment parmi les polymères réticulés décrits dans la demande EP-A-0295886.

Selon cette demande, ils sont obtenus par réaction d'addition et de réticulation d'au moins :

(a) un organopolysiloxane ayant au moins deux groupes alcényle inférieurs par molécule ;
(b) un organopolysiloxane ayant au moins deux atomes d'hydrogène liés à un atome de silicium par molécule ; et
(c) et un catalyseur du type platine.

Les groupes alcényle inférieurs sont notamment les groupes vinyl, allyl et propényl.

Le catalyseur au platine peut être par exemple l'acide chloroplatinique, les complexes contenant de l'acide chloroplatinique et le platine supporté par un support approprié.

Les organopolysiloxanes élastomères associés à une phase grasse selon l'invention peuvent être choisis aussi parmi ceux décrits dans le brevet US-A-5266321.

Selon ce brevet, ils sont choisis notamment parmi :

i) les polyorganopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;
ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 % mol quand l'organopolysiloxane est non-cyclique et entre 1 et 50 % mol lorsque l'organopolysiloxane est cyclique.

La phase grasse associée à l'organopolysiloxane solide élastomère est constituée d'au moins une huile hydrocarbonée et/ou d'au moins une huile siliconée.

Les huiles hydrocarbonées utilisées selon l'invention en association avec l'organopolysiloxane élastomère sont choisies parmi les huiles d'origine animale, les huiles d'origine végétale, les huiles de synthèse telles que l'isoparaffine hydrogénée, les esters et les éthers de synthèse et leurs mélanges.

Les huiles siliconées utilisées selon l'invention en association avec l'organopolysiloxane élastomère sont choisies de préférence parmi les polysiloxanes linéaires liquides ou pâteux à température ambiante tels que les alkylpolysiloxanes, les alkylphénylpolysiloxanes, alkylpolydiméthylsiloxane et les polysiloxanes cycliques tels que octaméthylcyclopentasiloxane, décaméthylcyclopentasiloxane; ou leurs mélanges.

De façon préférentielle, l'organopolysiloxane est présent dans le mélange organopolysiloxane élastomère / phase grasse sous forme d'un gel homogène, l'organopolysiloxane ayant une concentration allant de 3 à 80 % en poids.

Le gel résultant de cette association et contenant les nanopigments peut être utilisé tel quel et constituer lui-même une composition pour le soin ou le maquillage. Il peut également être incorporé dans une composition pour le soin, le traitement ou le maquillage.

Dans les compositions selon l'invention, l'organopolysiloxane élastomère est présent en une quantité allant de 0,3 à 60 %, et de préférence de 5 à 30 % du poids total de la composition.

Selon l'invention, les nanopigments peuvent être traités ou non traités en surface et peuvent être choisis notamment parmi les nanopigments d'oxydes de titane (amorphe ou cristallisé sous forme rutile et/ou anatase), de fer, de zirconium, de zinc ou de cérium.

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par:

- la silice et l'alumine tels que les produits 《 Microtitanium Dioxide MT 500 SA 》 et 《 Microtitanium Dioxide MT 100 SA 》 de la société TAYCA, et les produits 《 Tioveil Fin 》, 《 Tioveil OP 》, 《 Tioveil MOTG 》 et 《 Tioveil IPM 》 de la société TIOXIDE,
- l'alumine et le stéarate d'aluminium tels que le produit 《 Microtitanium Dioxide MT 100 T 》 de la société TAYCA,
- l'alumine et le laurate d'aluminium tels que le produit 《 Microtitanium Dioxide MT 100 S 》 de la société TAYCA,
- des oxydes de fer et le stéarate de fer tels que le produit 《 Microtitanium Dioxide MT 100 F 》 de la société TAYCA,
- la silice, l'alumine et la silicone tels que les produits 《 Microtitanium Dioxide MT 100 SAS 》, 《 Microtitanium Dioxide MT 600 SAS 》 et 《 Microtitanium Dioxide MT 500 SAS 》 de la société TAYCA,
- l'hexamétaphosphate de sodium tels que le produit 《 Microtitanium Dioxide MT 150 W 》 de la société TAYCA,
- l'octyltriméthoxysilane tels que le produit 《 T-805 》 de la société DEGUSSA,
- l'alumine et l'acide stéarique tels que le produit 《 UVT-M160 》 de la société KEMIRA,
- l'alumine et la glycérine tels que le produit 《 UVT-M212 》 de de la société KEMIRA,
- l'alumine et la silicone tels que le produit 《 UVT-M262 》 de de la société KEMIRA.

Les oxydes de titane non traités peuvent par exemple être ceux vendus par la société TAYCA sous les dénominations commerciales 《 Microtitanium Dioxide MT 500 B 》 ou 《 Microtitanium Dioxide MT 600 B 》.

Les oxydes de zinc non traités peuvent par exemple être ceux vendus par la société SUMITOMO sous la dénomination 《 Ultra Fine Zinc Oxide Powder 》, par la société PRESPERSE sous la dénomination 《 Finex 25 》, par la société IKEDA sous la dénomination 《 MZO-25 》 ou par la société SUNSMART sous la dénomination 《 Z-COTE 》. Les oxydes de zinc traités peuvent par exemple être ceux vendus par la société SUNSMART sous la dénomination 《 Z-COTE HP 1 》.

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Les nanopigments d'oxydes métalliques peuvent être présents dans les compositions selon l'invention dans une proportion allant de 0,5 à 30 % de préférence de 2 à 20 % du poids total de la composition.

Selon une réalisation préférée de l'invention, les compositions de l'invention, de préférence cosmétiques ou dermatologiques, contiennent un milieu physiologiquement acceptable, c'est-à-dire compatible avec la peau, le cuir chevelu et les cheveux, et peuvent être utilisées comme compositions protectrices de l'épiderme humain ou des cheveux contre les rayons ultraviolets, comme compositions antisolaires ou comme produits de maquillage, de traitement ou de soin de la peau.

Les compositions obtenues sont faciles à étaler et apportent des effets différents selon le type de nanopigments utilisés. Ainsi, les compositions contenant des nanotitanes apportent un léger effet opalescent sur la peau et éclaircissent le teint. Par ailleurs, les nanopigments d'oxyde de fer donnent un maquillage lumineux et naturel.

Les compositions de l'invention peuvent se présenter sous toutes les formes galéniques normalement utilisées pour une application topique telles que les solutions, les gels aqueux ou hydroalcooliques, les émulsions, notamment huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques. Les compositions de l'invention peuvent se présenter sous forme d'une crème, d'une pommade, d'une lotion, d'un lait ou d'un sérum.

Selon une forme préférée de réalisation de l'invention, les compositions sont anhydres.

Les compositions de l'invention peuvent contenir également tous les adjuvants classiquement utilisés dans le domaine cosmétique ou dermatologique, dans des concentrations habituelles. Ces adjuvants sont en particulier choisis parmi les gélifiants, les conservateurs, les opacifiants, les émulsionnants, les coémulsionnants, les neutralisants, les parfums et leurs solubilisants ou peptisants, les matières colorantes, les charges, ainsi que les actifs lipophiles ou hydrophiles et les filtres chimiques. Ces adjuvants sont présents dans des quantités allant de préférence de 0,01 à 30 % du poids de la composition.

Bien entendu, ces adjuvants doivent être de nature et en concentration telles qu'ils ne diminuent pas l'indice de protection de la composition et ne déstabilisent pas cette dernière.

En particulier, il est possible d'adjoindre aux compositions selon l'invention des filtres chimiques organiques UVA et/ou UVB, les filtres organiques UVA en vue de compléter l'effet protecteur apporté par les nanopigments et les filtres UVB en vue d'éviter les coups de soleil lors de l'exposition des utilisateurs de cette composition aux rayons solaires.

Les produits de maquillage contiennent généralement des charges. Par charges, on entend des matériaux naturels ou synthétiques dont la fonction principale consiste à modifier les propriétés physico-chimiques (rhéologiques, mécaniques, optiques) et/ou cosmétiques d'une composition. Les charges sont incolores ou plus ou moins blanches à l'état sec. Elles sont presque transparentes lorsque dispersées dans un liant. Parmi les charges, on peut citer le talc qui est un silicate de magnésium hydraté, utilisé sous forme de particules généralement de dimensions inférieures à 40 μm ; le talc possède des propriétés absorbantes de l'humidité et est utilisé surtout en raison de son toucher onctueux. Ces charges sont présentes dans des quantités allant de préférence de 0,01 à 40 % du poids de la composition.

Les compositions selon l'invention permettent une bonne protection, et notamment une bonne photoprotection de la peau et/ou des cheveux, et par voie de conséquence, ont une action sur le vieillissement photo-induit de la peau ainsi que sur les rides et/ou ridules de la peau induites par le rayonnement solaire.

Aussi, l'invention se rapporte également à l'utilisation cosmétique de la composition telle que définie ci-dessus pour protéger la peau et/ou les cheveux et/ou pour lutter contre le vieillissement de la peau photo-induit et/ou contre les rides et/ou les ridules de la peau induites par un rayonnement solaire.

L'invention se rapporte encore à l'utilisation de la composition telle que définie ci-dessus pour la fabrication d'une composition dermatologique destinée à protéger la peau et/ou les cheveux et/ou à lutter contre le vieillissement de la peau photo-induit et/ou contre les rides et/ou les ridules de la peau induites par un rayonnement solaire.

Elle se rapporte aussi à un procédé cosmétique et/ou dermatologique pour la photoprotection de la peau et/ou des cheveux, caractérisé par le fait qu'on applique sur la peau et/ou les cheveux une quantité efficace de la composition telle que définie ci-dessus.

L'invention a enfin pour objet un procédé cosmétique et/ou dermatologique pour lutter contre le vieillissement photo-induit et/ou contre les rides et/ou les ridules de la peau induites par un rayonnement solaire, caractérisé par le fait qu'on applique sur la peau une quantité efficace de la composition telle que définie ci-dessus.

L'exemple qui suit sert à illustrer l'invention sans toutefois présenter un caractère limitatif. Dans cet exemple, la composition est donnée en % pondéral.

**Exemple : Baume anhydre pour le visage**

| Phase A : | |
|---|---|
| - Polydiméthylorganosiloxane partiellement réticulé/ polydiméthylsiloxane 6 cst (vendu sous le nom KSG 16 par la société KOSE) | 20 % |
| - Cyclopentadiméthylsiloxane | 28,95 % |
| - Hectorite modifiée (Miglyol Gel B vendu par la société Huls) (gélifiant) | 30 % |
| Phase B : | |
| - Nano-oxyde de titane (Microtitanium Dioxide MT100 T vendu par la société Tayca) | 10 % |
| - Isoparaffine hydrogénée (Polysynlane vendu par la société Nippon Oils Fats) | 9 % |
| Phase C : | |
| - Talc | 6 % |

Mode opératoire :

Les phases A et B sont préparées séparément. La phase A est homogénéisée à température ambiante. Pour préparer la phase B, on saupoudre progressivement les nano-oxydes de titane dans l'huile sous agitation ; on poursuit l'agitation pendant 30 minutes, puis on broie le mélange. On ajoute la phase B à la phase A. On homogénéise et on saupoudre la phase C dans le mélange.

Le baume obtenu de couleur blanche, a un indice de protection d'environ 14,4 et peut être utilisé sur la peau du visage, comme crème de jour ou comme base de teint pour corriger les imperfections du teint avant l'application du maquillage.

On a comparé la protection contre les UVA, apportée par ce baume à celle obtenue en utilisant des émulsions eau-dans-huile (E/H) et huile-dans-eau (H/E) contenant une quantité identique des mêmes nanotitanes.

Comme indiqué ci-dessus, les indices de protection IP sont déterminés selon la méthode PPD. Les tests sont réalisés sur les dos des sujets où sont délimitées des zones cutanées, ayant une surface d'environ 7,5 cm$^2$. Le produit à tester n'est appliqué que sur certaines zones. 15 minutes après application du produit, toutes les zones (celles ayant reçu du produit et les autres) sont soumises à l'exposition d'UVA produits par une lampe à arc xénon de 150 W munie de filtres WG 335 et UG 11, délivrant des doses de rayonnement ultraviolet de 5 à 38 joules/cm$^2$, ces doses étant en progression géométrique de 50 %.

On détermine la dose pigmentante minimale (D.P.M.), c'est-à-dire la dose provoquant la première réaction de pigmentation perceptible sans ambiguïté à l'oeil et homogène, pour une zone protégée par le produit à étudier (D.P.M. protégée) et pour une zone où aucun produit n'a été appliqué (D.P.M. non protégée).

L'indice de protection est le rapport entre D.P.M. protégée et D.P.M. non protégée :

$$IP = \frac{D.P.M.\ protégée}{D.P.M.\ non\ protégée}$$

Les résultats ont été les suivants :

| Composition | IP obtenu |
|---|---|
| Exemple selon l'invention | 18,34 |
| Emulsion E/H | 17,06 |
| Emulsion H/E | 13,48 |

Ces résultats montrent que l'addition de l'organopolysiloxane élastomère selon l'invention permet d'améliorer notablement l'indice de protection apporté par les nanopigments. La différence d'indice de protection entre l'émulsion E/H et l'exemple selon l'invention est significative.

**Revendications**

**1.** Composition à application topique contenant des nanopigments d'au moins un oxyde métallique, la taille moyenne des particules élémentaires desdits nanopigments allant de 5 à 100 nm, caractérisée en ce qu'elle contient en outre un organopolysiloxane solide élastomère associé à une phase grasse, en vue d'améliorer l'indice de protection apporté par les nanopigments.

**2.** Composition selon la revendication 1, caractérisée par le fait que l'organopolysiloxane est partiellement ou totalement réticulé.

**3.** Composition selon l'une quelconque des revendications 1 ou 2, caractérisée par le fait que l'organopolysiloxane élastomère associé à une phase grasse est inclus dans au moins une huile hydrocarbonée et/ou au moins une huile siliconée pour former un gel.

**4.** Composition selon l'une quelconque des revendications précédentes, caractérisée par le fait que l'organopolysiloxane élastomère associé à une phase grasse est obtenu par réaction d'addition et de réticulation d'au moins :

(a) un organopolysiloxane ayant au moins deux groupes alcenyle inférieurs par molécule ;

(b) un organopolysiloxane ayant au moins deux atomes d'hydrogène liés à un atome de silicium par molécule ; et

(c) et un catalyseur du type platine.

5. Composition selon l'une quelconque des revendications 1 à 3, caractérisée par le fait que l'organopolysiloxane est choisi parmi :

i) les polyorganopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 % mol quand l'organopolysiloxane est non-cyclique et entre 1 et 50 % moi lorsque l'organopolysiloxane est cyclique.

6. Composition selon l'une quelconque des revendications 3 à 5, caractérisée par le fait que les huiles hydrocarbonées utilisées en association avec l'organopolysiloxane élastomère sont choisies parmi les huiles d'origine animale, les huiles d'origine végétale, les huiles de synthèse, les esters et éthers de synthèse et leurs mélanges.

7. Composition selon l'une quelconque des revendications 3 à 6, caractérisée par le fait que les huiles siliconées utilisées en association avec l'organopolysiloxane élastomère sont choisies parmi les polysiloxanes linéaires liquides ou pâteux à température ambiante, les polysiloxanes cycliques ou leurs mélanges.

8. Composition selon l'une quelconque des revendications précédentes, caractérisée par le fait que l'organopolysiloxane solide élastomère associé à une phase grasse est présent dans le mélange organopolysiloxane élastomère / phase grasse pour former un gel homogène, à une concentration allant de 3 à 80 % en poids.

9. Composition selon l'une quelconque des revendications précédentes, caractérisée par le fait que l'organopolysiloxane solide élastomère associé à une phase grasse est présent en une quantité allant de 0,3 à 60 % du poids total de la composition.

10. Composition selon l'une quelconque des revendications précédentes, caractérisée par le fait que les particules élémentaires des nanopigments ont une dimension de 10 à 60 nm.

11. Composition selon l'une quelconque des revendications précédentes, caractérisée par le fait que les nanopigments sont choisis parmi les nanopigments d'oxydes de titane, de fer, de zirconium, de zinc ou de cérium.

12. Composition selon l'une quelconque des revendications précédentes, caractérisée par le fait que les nanopigments sont enrobés.

13. Composition selon l'une quelconque des revendications précédentes, caractérisée par le fait que les nanopigments sont introduits sous forme de pâte pigmentaire.

14. Composition selon l'une quelconque des revendications précédentes, caractérisée par le fait que les nanopigments sont présents en une proportion allant de 0,5 à 30 % du poids total de la composition.

15. Composition selon l'une quelconque des revendications précédentes, caractérisée par le fait qu'elle contient, en outre, au moins une charge.

16. Composition selon l'une quelconque des revendications précédentes, caractérisée par le fait qu'elle constitue une composition pour le soin et/ou le maquillage de la peau.

17. Composition selon l'une quelconque des revendications précédentes, caractérisée par le fait qu'elle est anhydre.

**18.** Composition solaire et/ou de maquillage de la peau, caractérisée en ce qu'elle contient une composition selon l'une quelconque des revendications précédentes.

**19.** Utilisation cosmétique de la composition selon l'une quelconque des revendications précédentes, pour protéger la peau et/ou les cheveux et/ou pour lutter contre le vieillissement de la peau photo-induit et/ou contre les rides et/ou les ridules de la peau induites par un rayonnement solaire.

**20.** Utilisation de la composition selon l'une quelconque des revendications 1 à 18, pour la fabrication d'une composition dermatologique destinée à protéger la peau et/ou les cheveux et/ou à lutter contre le vieillissement de la peau photo-induit et/ou contre les rides et/ou les ridules de la peau induites par un rayonnement solaire.

**21.** Utilisation d'un organopolysiloxane solide élastomère associé à une phase grasse, dans une composition à application topique contenant des nanopigments, la taille moyenne des particules élémentaires desdits nanopigments allant de 5 à 100 nm, pour améliorer la stabilité de la dite composition et/ou conférer à la dite composition un indice de protection amélioré.

**22.** Procédé cosmétique pour la photoprotection de la peau et/ou des cheveux, caractérisé par le fait qu'on applique sur la peau et/ou les cheveux une quantité efficace de la composition selon l'une quelconque des revendications 1 à 18.

**23.** Procédé cosmétique pour lutter contre le vieillissement photo-induit et/ou contre les rides et/ou les ridules de la peau induites par un rayonnement solaire, caractérisé par le fait qu'on applique sur la peau une quantité efficace de la composition selon l'une quelconque des revendications 1 à 18.

## Claims

**1.** Composition for topical application containing nanopigments of at least one metal oxide, the average size of the elementary particles of the said nanopigments ranging from 5 to 100 nm, characterized in that it also contains a solid elastomeric polyorganosiloxane combined with a fatty phase, in order to improve the protection factor provided by the nanopigments.

**2.** Composition according to Claim 1, characterized in that the polyorganosiloxane is partially or totally crosslinked.

**3.** Composition according to either of Claims 1 and 2, characterized in that the elastomeric polyorganosiloxane combined with a fatty phase is included in at least one hydrocarbon oil and/or at least one silicone oil in order to form a gel.

**4.** Composition according to any one of the preceding claims, characterized in that the elastomeric polyorganosiloxane combined with a fatty phase is obtained by addition reaction and crosslinking of at least:

(a) one polyorganosiloxane having at least two lower alkenyl groups per molecule;
(b) one polyorganosiloxane having at least two hydrogen atoms linked to a silicon atom per molecule; and
(c) one platinum-type catalyst

**5.** Composition according to any one of Claims 1 to 3, characterized in that the polyorganosiloxane is chosen from:

i) polyorganopolysiloxanes comprising units $R_2SiO$ and $RSiO_{1.5}$ and optionally units $R_3SiO_{0.5}$ and/or $SiO_2$ in which the radicals R, independently of each other, denote a hydrogen, an alkyl such as methyl, ethyl or propyl, an aryl such as phenyl or tolyl, or an unsaturated aliphatic group such as vinyl, and in which the weight ratio of the units $R_2SiO$ to the units $RSiO_{1.5}$ ranges from 1/1 to 30/1;
ii) insoluble polyorganopolysiloxanes which can be swollen in silicone oil, obtained by addition of an organohydrogenopolysiloxane (1) and a polyorganosiloxane (2) having unsaturated aliphatic groups such that the amount of hydrogen or of unsaturated aliphatic groups in (1) and (2) respectively is between 1 and 20 mol% when the polyorganosiloxane is acyclic and between 1 and 50 mol% when the polyorganosiloxane is cyclic.

**6.** Composition according to any one of Claims 3 to 5, characterized in that the hydrocarbon oils used in combination with the elastomeric polyorganosiloxane are chosen from oils of animal origin, oils of plant origin, synthetic oils, synthetic esters and ethers, and mixtures thereof.

7. Composition according to any one of Claims 3 to 6, characterized in that the silicone oils used in combination with the elastomeric polyorganosiloxane are chosen from linear polysiloxanes which are liquid or pasty at room temperature, cyclic polysiloxanes or mixtures thereof.

8. Composition according to any one of the preceding claims, characterized in that the solid elastomeric polyorganosiloxane combined with a fatty phase is present in the elastomeric polyorganosiloxane/fatty phase mixture in order to form a homogeneous gel, at a concentration ranging from 3 to 80% by weight.

9. Composition according to any one of the preceding claims, characterized in that the solid elastomeric polyorganosiloxane combined with a fatty phase is present in an amount ranging from 0.3 to 60% of the total weight of the composition.

10. Composition according to any one of the preceding claims, characterized in that the elementary particles of the nanopigments are from 10 to 60 nm in size.

11. Composition according to any one of the preceding claims, characterized in that the nanopigments are chosen from nanopigments of titanium oxide, of iron oxide, of zirconium oxide, of zinc oxide or of cerium oxide.

12. Composition according to any one of the preceding claims, characterized in that the nanopigments are coated.

13. Composition according to any one of the preceding claims, characterized in that the nanopigments are introduced in the form of a pigmentary paste.

14. Composition according to any one of the preceding claims, characterized in that the nanopigments are present in a proportion ranging from 0.5 to 30% of the total weight of the composition.

15. Composition according to any one of the preceding claims, characterized in that it also contains at least one filler.

16. Composition according to any one of the preceding claims, characterized in that it constitutes a composition to care for and/or make up the skin.

17. Composition according to any one of the preceding claims, characterized in that it is anhydrous.

18. Antisun and/or make-up composition for the skin, characterized in that it contains a composition according to any one of the preceding claims.

19. Cosmetic use of the composition according to any one of the preceding claims for protecting the skin and/or the hair and/or for combating photoinduced ageing of the skin and/or for combating wrinkles and/or fine lines on the skin which are induced by solar radiation.

20. Use of the composition according to any one of Claims 1 to 18 for the manufacture of a dermatological composition intended to protect the skin and/or the hair and/or to combat photoinduced ageing of the skin and/or to combat wrinkles and/or fine lines on the skin which are induced by solar radiation.

21. Use of a solid elastomeric polyorganosiloxane combined with a fatty phase, in a composition for topical application containing nanopigments, the average size of the elementary particles of the said nanopigments ranging from 5 to 100 nm, in order to improve the stability of the said composition and/or to give the said composition an improved protection factor.

22. Cosmetic process for the photoprotection of the skin and/or the hair, characterized in that an effective amount of the composition according to any one of Claims 1 to 18 is applied to the skin and/or the hair.

23. Cosmetic process for combating photoinduced ageing and/or for combating wrinkles and/or fine lines on the skin which are induced by solar radiation, characterized in that an effective amount of the composition according to any one of Claims 1 to 18 is applied to the skin.

**Patentansprüche**

1. Zusammensetzung zur topischen Anwendung, die Nanopigmente mindestens eines Metalloxids enthält, wobei die mittlere Größe der Elementarpartikel dieser Nanopigmente im Bereich von 5 bis 100 nm liegt, dadurch gekennzeichnet, daß sie zur Erhöhung des Lichtschutzfaktors, der mit den Nanopigmenten erzielt wird, außerdem ein elastomeres festes Polyorganosiloxan enthält, das mit einer Fettphase kombiniert ist.

2. Zusammensetzung nach Anspruch 1, dadurch gekennzeichnet, daß das Polyorganosiloxan teilweise oder vollständig vernetzt ist.

3. Zusammensetzung nach Anspruch 1 oder 2, dadurch gekennzeichnet, daß das elastomere Polyorganosiloxan, das mit einer Fettphase kombiniert ist, zur Bildung eines Gels in mindestens einem Kohlenwasserstofföl und/oder mindestens einem Siliconöl eingeschlossen ist.

4. Zusammensetzung nach einem der vorhergehenden Ansprüche, dadurch gekennzeichnet, daß das elastomere Polyorganosiloxan, das mit einer Fettphase kombiniert ist, hergestellt ist durch Additions- und Vernetzungsreaktion mindestens

   (a) eines Polyorganosiloxans mit mindestens zwei niederen Alkenylgruppen pro Molekül,
   (b) eines Polyorganosiloxans mit mindestens zwei Wasserstoffatomen, die mit einem Siliciumatom verbunden sind, pro Molekül und
   (c) in Gegenwart eines Katalysators vom Platin-Typ hergestellt ist.

5. Zusammensetzung nach einem der Ansprüche 1 bis 3, dadurch gekennzeichnet, daß das Polyorganosiloxan ausgewählt ist unter:

   i) Polyorganopolysiloxanen, die Einheiten $R_2SiO$ und $RSiO_{1,5}$ und gegebenenfalls Einheiten $R_3SiO_{0,5}$ und/oder $SiO_2$ enthalten, worin die Reste R unabhängig Wasserstoff, Alkyl wie z.B. Methyl, Ethyl oder Propyl, Aryl, wie z.B. Phenyl oder Tolyl, eine ungesättigte aliphatische Gruppe, wie z.B. Vinyl, bedeuten, wobei das Gewichtsverhältnis der Einheiten $R_2SiO$ zu den Einheiten $RSiO_{1,5}$ im Bereich von 1/1 bis 30/1 variiert;
   ii) Polyorganopolysiloxanen, die in Siliconöl unlöslich und quellfähig sind, die durch die Additionreaktion eines Polyorganosiloxans, das siliciumgebundenen Wasserstoff aufweist (1), mit einem Polyorganosiloxan (2), das ungesättigte aliphatische Gruppen aufweist, hergestellt werden, die in solchen Mengenanteilen verwendet werden, daß die Menge des Wasserstoffs oder der ungesättigten aliphatischen Gruppen in (1) bzw. (2) im Bereich von 1 bis 20 Mol-% liegt, wenn das Polyorganosiloxan nicht cyclisch ist, und im Bereich von 1 bis 50 Mol-% liegt, wenn das Polyorganosiloxan cyclisch ist.

6. Zusammensetzung nach einem der Ansprüche 3 bis 5, dadurch gekennzeichnet, daß die in Kombination mit dem elastomeren Polyorganosiloxan verwendeten Kohlenwasserstofföle unter Ölen tierischer Herkunft, Ölen pflanzlicher Herkunft, synthetischen Ölen, synthetischen Estern und Ethern und deren Gemischen ausgewählt sind.

7. Zusammensetzung nach einem der Ansprüche 3 bis 6, dadurch gekennzeichnet, daß die in Kombination mit dem elastomeren Polyorganosiloxan verwendeten Siliconöle unter geradkettigen, bei Umgebungstemperatur flüssigen oder pastösen Polysiloxanen, cyclischen Polysiloxanen und deren Gemischen ausgewählt sind.

8. Zusammensetzung nach einem der vorhergehenden Ansprüche, dadurch gekennzeichnet, daß das elastomere feste Polyorganosiloxan, das mit einer Fettphase kombiniert ist, in dem Gemisch aus elastomerem Polyorganosiloxan und Fettphase zur Bildung eines homogenen Gels in einer Konzentration von 3 bis 80 Gew.-% enthalten ist.

9. Zusammensetzung nach einem der vorhergehenden Ansprüche, dadurch gekennzeichnet, daß das elastomere feste Polyorganosiloxan, das mit einer Fettphase kombiniert ist, in einer Menge von 0,3 bis 60 % des Gesamtgewichts der Zusammensetzung enthalten ist.

10. Zusammensetzung nach einem der vorhergehenden Ansprüche, dadurch gekennzeichnet, daß die Elementarpartikel der Nanopigmente eine Größe von 10 bis 60 nm aufweisen.

11. Zusammensetzung nach einem der vorhergehenden Ansprüche, dadurch gekennzeichnet, daß die Nanopigmente unter den Nanopigmenten der Oxide von Titan, Eisen, Zirconium, Zink und Cer ausgewählt sind.

12. Zusammensetzung nach einem der vorhergehenden Ansprüche, dadurch gekennzeichnet, daß die Nanopigmente umhüllt sind.

13. Zusammensetzung nach einem der vorhergehenden Ansprüche, dadurch gekennzeichnet, daß die Nanopigmente in Form einer Pigmentpaste zugegeben sind.

14. Zusammensetzung nach einem der vorhergehenden Ansprüche, dadurch gekennzeichnet, daß die Nanopigmente in einem Anteil von 0,5 bis 30 % des Gesamtgewichts der Zusammensetzung enthalten sind.

15. Zusammensetzung nach einem der vorhergehenden Ansprüche, dadurch gekennzeichnet, daß sie außerdem mindestens einen Füllstoff enthält.

16. Zusammensetzung nach einem der vorhergehenden Ansprüche, dadurch gekennzeichnet, daß sie eine Zusammensetzung zur Pflege und/oder zum Schminken der Haut bildet.

17. Zusammensetzung nach einem der vorhergehenden Ansprüche, dadurch gekennzeichnet, daß sie wasserfrei ist.

18. Zusammensetzung für den Sonnenlichtschutz und/oder zum Schminken der Haut, dadurch gekennzeichnet, daß sie eine Zusammensetzung nach einem der vorhergehenden Ansprüche enthält.

19. Kosmetische Verwendung der Zusammensetzung nach einem der vorhergehenden Ansprüche zum Schutz der Haut und/oder der Haare und/oder zur Bekämpfung der lichtbedingten Hautalterung und/oder zur Bekämpfung der Hautfalten und/oder Hautfältchen, die durch Licht hervorgerufen werden.

20. Verwendung der Zusammensetzung nach einem der Ansprüche 1 bis 18 zur Herstellung einer dermatologischen Zusammensetzung, die zum Schutz der Haut und/oder der Haare und/oder zur Bekämpfung der lichtinduzierten Hautalterung und/oder zur Bekämpfung der Hautfalten und/oder Hautfältchen, die durch Licht hervorgerufen werden, vorgesehen ist.

21. Verwendung eines elastomeren festen Polyorganosiloxans, das mit einer Fettphase kombiniert ist, in einer Zusammensetzung zur topischen Anwendung, die Nanopigmente enthält, wobei die mittlere Größe der Elementarpartikel dieser Nanopigmente im Bereich von 5 bis 100 nm liegt, zur Verbesserung der Stabilität dieser Zusammensetzung und/oder zur Erhöhung des Lichtschutzfaktors dieser Zusammensetzung.

22. Kosmetisches Verfahren für den Lichtschutz der Haut und/oder der Haare, dadurch gekennzeichnet, daß auf die Haut und/oder die Haare eine wirksame Menge der Zusammensetzung nach einem der Ansprüche 1 bis 18 aufgetragen wird.

23. Kosmetische Zusammensetzung zur Bekämpfung der lichtinduzierten Hautalterung und/oder zur Bekämpfung der Hautfalten und/oder Hautfältchen, die durch Licht hervorgerufen werden, dadurch gekennzeichnet, daß auf die Haut eine wirksame Menge der Zusammensetzung nach einem der Ansprüche 1 bis 18 aufgetragen wird.